(19) Europäisches Patentamt
European Patent Office
Office européen des brevets

(11) **EP 3 418 373 B1**

(12) **EUROPEAN PATENT SPECIFICATION**

(45) Date of publication and mention
of the grant of the patent:
**23.02.2022 Bulletin 2022/08**

(21) Application number: **17752948.4**

(22) Date of filing: **31.01.2017**

(51) International Patent Classification (IPC):
**B03C 5/00** (2006.01)   **B03C 5/02** (2006.01)
**C12M 1/00** (2006.01)   **C12N 13/00** (2006.01)
**C12Q 1/02** (2006.01)

(52) Cooperative Patent Classification (CPC):
**B03C 5/005; B03C 5/026; C12M 47/02;**
**C12N 13/00;** B03C 2201/26; C12Q 1/02

(86) International application number:
**PCT/JP2017/003301**

(87) International publication number:
**WO 2017/141685 (24.08.2017 Gazette 2017/34)**

(54) **SEPARATION DEVICE**

**TRENNVORRICHTUNG**

**DISPOSITIF DE SÉPARATION**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.02.2016   JP 2016026673**

(43) Date of publication of application:
**26.12.2018 Bulletin 2018/52**

(73) Proprietors:
• **AFI Corporation**
  **Kyoto-shi, Kyoto 606-8501 (JP)**
• **Kyoto University**
  **Kyoto-shi, Kyoto 606-8501 (JP)**

(72) Inventors:
• **WAKIZAKA, Yoshikazu**
  **Kyoto-shi,**
  **Kyoto 606-8501 (JP)**
• **TAKANO, Masayo**
  **Kyoto-shi,**
  **Kyoto 606-8501 (JP)**
• **ITOI, Takayuki**
  **Kyoto-shi,**
  **Kyoto 606-8501 (JP)**
• **ENJOJI, Takaharu**
  **Kyoto-shi,**
  **Kyoto 606-8501 (JP)**

• **TOI, Masakazu**
  **Kyoto-shi,**
  **Kyoto 606-8501 (JP)**
• **NISHIMURA, Tomomi**
  **Kyoto-shi,**
  **Kyoto 606-8501 (JP)**

(74) Representative: **Eisenführ Speiser**
**Patentanwälte Rechtsanwälte PartGmbB**
**Postfach 10 60 78**
**28060 Bremen (DE)**

(56) References cited:
**WO-A1-01/05512**       **WO-A1-2014/155932**
**WO-A2-2012/054904**    **WO-A2-2013/019810**
**JP-A- 2012 098 063**   **US-A1- 2007 152 206**
**US-A1- 2008 283 401**  **US-B1- 6 280 590**
**US-B1- 6 749 736**

• **VOLDMAN, J. et al.: "Design and analysis of
extruded quadrupolar dielectrophoretic traps",
Journal of Electrostatics, vol. 57, 2003, pages
69-90, XP004391248,**
• **MARTINEZ-DUARTE , RODRIGO: "A novel
approach to dielectrophoresis using carbon
electrodes", Electrophoresis, vol. 32, 2011, pages
2385-2392, XP055533930,**

## Description

Technical Field

The present invention relates to a separation device having the features of preamble of claim 1 that separates dielectric particles such as bacteria and cells.

Background Art

A device and method for manipulating particles using dielectrophoresis are disclosed in US 2007/152206 A1. The device comprises a chamber comprising an inlet port, an outlet port, and metal post electrodes, and a power supply, wherein the metal post electrodes are arranged in at least two rows in a vertical position with respect to the flow of fluids, each row comprises at least two metal post electrodes, each odd row is wired to a metal pad through a metal line, and each even row is wired to another metal pad through a metal line, and the power supply is connected to the metal pads.

US 2008/283401 A1 discloses methods, devices, and systems for separating a time-varying flow of disparate liquid-suspended particles through a channel using dielectrophoresis and field-flow fractionation.

According to WO 2013/019810 A2 a processing method and apparatus uses at least one electric field applicator biased to produce a spatial-temporal electric field to affect a processing medium, suspended nano-objects or the substrate in processing, interacting with the dipole properties of the medium or particles to construct structure on the substrate. The apparatus may include a magnetic field, an acoustic field, an optical force, or other generation device. The processing may affect selective localized layers on the substrate or may control orientation of particles in the layers, control movement of dielectrophoretic particles or media, or cause suspended particles of different properties to follow different paths in the processing medium. Depositing or modifying a layer on the substrate may be carried out. Further, the processing medium and electrical bias may be selected to prepare at least one layer on the substrate for bonding the substrate to a second substrate, or to deposit carbon nanotubes with a controlled orientation on the substrate.

Techniques, devices and systems are described in WO 2012/054904 A2 for incorporating a printed circuit with a micro fluidic device and wirelessly powering the micro fluidic device. In one aspect, a microfluidic device includes a substrate with a fluidic channel to provide a path for a fluid with particles. The fluidic channel includes fluid inlet and outlet. A pair of electrodes near the inlet and the outlet guides the particles toward a center of the fluidic channel using negative-dielectrophoresis (DEP) effect in response to an alternating current (AC) frequency voltage received at the pairs of electrodes. Additional pairs of electrodes are disposed along a border of the fluidic channel between the pairs of electrodes near the inlet and the outlet of the fluidic channel to isolate a subpopulation of the particles using positive and negative DEP effects in response to AC voltages of different frequencies received at different ones of the additional pairs of electrodes.

WO 01/05512 A1 relates to a dielectrophoretic apparatus and method. In a dielectrophoretic cell having an array of electrodes and means to apply electrical signals to the electrodes, the electrodes comprise a planar array of serpentine or zig-zag electrodes with their curvatures in register. The serpentine electrodes may be sinusoidal, half sinusoidal, or elongated "C" in shape; the positions of maximum curvature of each serpentine or zig-zag electrode may be arranged in linear alignment, or along a curve. The cell may be used for stationary or travelling wave dielectrophoresis. Particles travelling in opposite directions in travelling wave dielectrophoresis can do so without interference, allowing "traffic control". Particles can be characterised and separated, and particles at high concentrations, or particles of different types, can be handled.

A separation method for separating dielectric particles such as bacteria and cells by using dielectrophoresis is known. For example, Patent Literature 1 discloses a separation method for separating bacteria in sample liquid using dielectrophoresis. In this separation method, electrodes are arranged in a flow channel through which the sample liquid flows, and the separation is performed by collecting the bacteria at the electrodes.

Citation List

PATENT DOCUMENT

Patent Literature 1: JP 2013-27366 A

Summary of Invention

TECHNICAL PROBLEMS

In the conventional method of separating dielectric particles, a microchannel for flowing dielectric particles is formed on a planar shaped (two-dimensional shaped) electrodes formed by photolithography or the like. Dielectrophoretic force drops sharply when away from the electrodes, so it works effectively only in the vicinity of the electrodes. Therefore, in the conventional separation method, the dielectrophoretic force is weak above the microchannel, and the microchannel is as thin as, for example, about 30 $\mu$m in height. Therefore, the effective volume of the separation treatment of the dielectric particles is small and the separation treatment capacity is low.

In addition, in the conventional separation method, since the dielectrophoretic force varies depending on the position of the dielectric particles in the height direction of the microchannel, the dielectric particles above the microchannel are hard to be captured and pass through, and may not be separated. Therefore, since the separation accuracy varies depending on the position of the dielectric particles in the height direction of the microchannel, the accuracy as a whole is low.

An object of the present invention is to provide a separation device capable of improving the capacity to separate dielectric particles.

SOLUTIONS TO PROBLEMS

A separation device according to the present invention is defined in claim 1.

ADVANTAGEOUS EFFECTS OF INVENTION

According to the present invention, the capacity to separate dielectric particles can be improved.

Brief Description of Drawings

Fig. 1 is a diagram showing a configuration of a separation device according to a first embodiment.
Fig. 2 is a perspective view showing a configuration of an electrode unit of the separation device according to the first embodiment.
Fig. 3 is a plan view showing the configuration of the electrode unit of the separation device according to the first embodiment.
Fig. 4 is a diagram showing an operation of the separation device according to the first embodiment.
Fig. 5 is a diagram showing the operation of the separation device according to the first embodiment.
Fig. 6 is a diagram showing an operation of a separation device according to a second embodiment.
Fig. 7 is a perspective view showing a configuration of an electrode unit of a separation device according to a third embodiment.
Fig. 8 is a plan view showing the configuration of the electrode unit of the separation device according to the third embodiment.
Fig. 9 is a diagram showing an operation of the separation device according to the third embodiment.
Fig. 10 is a diagram showing the operation of the separation device according to the third embodiment.
Fig. 11 is a plan view showing a configuration of an electrode unit of a separation device according to a fourth embodiment.
Fig. 12 is a diagram showing an operation of the separation device according to the fourth embodiment.

Embodiment of the Invention

Embodiments of a separation device that separates dielectric particles will be described below with reference to the accompanying drawings. Of the embodiments, only the third and the fourth embodiment fall within the scope of the claims, whereas the first and the second embodiment are provided for being useful for understanding the invention.

0. Outline of Dielectrophoresis

Before describing the present embodiments, outline of dielectrophoresis will be described. When electrodes are arranged in sample liquid containing dielectric particles such as bacteria and cells, and an AC voltage with frequency $\omega$ is supplied to the electrodes, dielectrophoretic force acts on the dielectric particles in the sample liquid. This dielectrophoretic force $F_{DEP}$ is expressed by the following equation:

$$F_{DEP} = 2\pi r^3 \varepsilon_m Re[K(\omega)]\nabla E^2 \ ...(1)$$

In equation (1) above, r is a radius of the dielectric particle, $\varepsilon_m$ is a permittivity of the medium (solution) of the sample liquid, and E is an intensity of the electric field. Re[X] represents the real part of the complex number X. $K(\omega)$ is the Clausius-Mossotti factor and is expressed by the following equation:

$$K(\omega) = (\varepsilon_p{}^* - \varepsilon_m{}^*) / (\varepsilon_p{}^* + 2\varepsilon_m{}^*) \quad \ldots (2)$$

In Equation (2) above, $\varepsilon_p{}^* (= \varepsilon_p + \rho_p/(j(\omega)))$ is a complex permittivity of the particles ($\varepsilon_p$ is a permittivity (real part) of the particles, and $\rho_p$ is a conductivity of the particles). Further, $\varepsilon_m{}^* (= \varepsilon_m + \rho_m/(j\omega))$ is a complex permittivity of the surrounding medium ($\varepsilon_m$ is a permittivity (real part) of the surrounding medium, and $\rho_m$ is a conductivity of the surrounding medium).

When Re[K($\omega$)] > 0 in the above equation (1), a positive dielectrophoretic force $F_{DEP}$ (attracting force) acts on the particles with respect to the installation direction of the electrodes, and the particles are attracted to the vicinity of the electrodes and are captured by the electrode. On the other hand, when Re[K($\omega$)] < 0, a negative dielectrophoretic force $F_{DEP}$ (repulsive force) acts on the particles, and the particles repel the electrodes and are captured between the electrodes. When Re[K($\omega$)] = 0, the dielectrophoretic force does not act on the particles, and the particles are not captured by the electrodes. The frequency at Re[K($\omega$)] = 0, that is, the frequency at the boundary where the dielectrophoretic force $F_{DEP}$ changes from positive to negative or vice versa is called a crossover frequency (COF).

From the above, by appropriately setting the frequency $\omega$ of the AC voltage to be supplied to the electrodes, for example, while excluding particles other than the target particles, the target particles can be selectively captured by the electrodes and separated.

(First Embodiment)

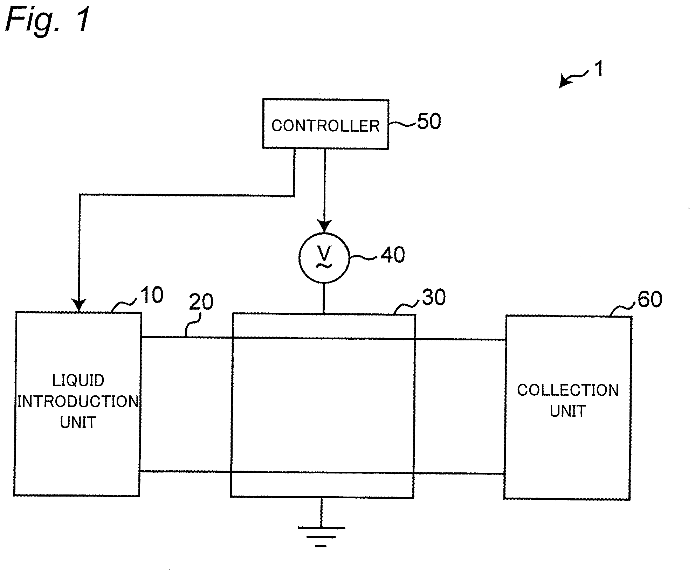

Hereinafter, the separation device according to a first embodiment will be described with reference to Figs. 1 to 5.

1. Configuration

1-1. Separation device

Fig. 1 is a diagram showing a configuration of a separation device according to the first embodiment. A separation device 1 shown in Fig. 1 separates target cells by using dielectrophoresis in a suspension containing the target cells and other cells. The separation device 1 includes a liquid introduction unit 10, a flow channel 20, an electrode unit 30, a power supply 40, a controller 50, and a collection unit 60.

The liquid introduction unit 10 introduces a suspension containing target cells and other cells and dielectrophoretic liquid (DEP liquid) into the flow channel 20. In addition, the liquid introduction unit 10 introduces an eluate for delivering out the target cells captured by the electrode unit 30 from the flow channel 20 into the flow channel 20. Introduction of each liquid by the liquid introduction unit 10 is controlled by the controller 50.

The flow channel 20 feeds the suspension liquid, the dielectrophoretic liquid, or the eluate introduced by the liquid introduction unit 10 in a predetermined direction (liquid flow direction).

The electrode unit 30 is arranged in the flow channel 20. The electrode unit 30 has a plurality of electrodes for causing the dielectrophoretic force to act on target cells flowing through the flow channel 20. Details of the electrode unit 30 will be described later.

The power supply 40 is composed of, for example, a function generator. Under the control of the controller 50, the power supply 40 generates an AC voltage with a predetermined frequency, and supplies it to electrodes of the electrode unit 30.

The controller 50 is composed of, for example, a personal computer. The controller 50 includes a storage unit such as an HDD and an SSD, and a controller such as a CPU, and the controller implements various functions by executing a program stored in the storage unit. The controller 50 may be constituted by a hardware circuit (ASIC, FPGA, etc.) such as a dedicated electronic circuit or a reconfigurable electronic circuit. The function of the controller 50 may be implemented by cooperation of hardware and software, or may be implemented only by hardware (electronic circuit).

The controller 50 controls the start and stop of the output of the AC voltage by the power supply 40, the magnitude of the AC voltage, and the frequency of the AC voltage. In addition, the controller 50 controls introduction of the suspension and the dielectrophoretic liquid by the liquid introduction unit 10, and introduction of the eluate.

The collection unit 60 collects target cells. In addition, the collection unit 60 discards cells other than the target cells.

1-2. Electrode Unit

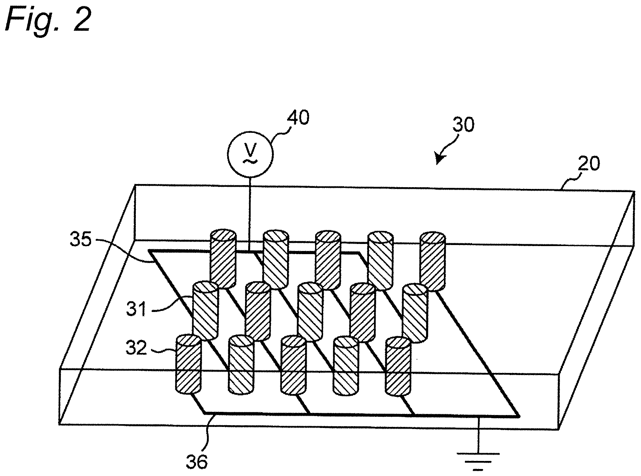

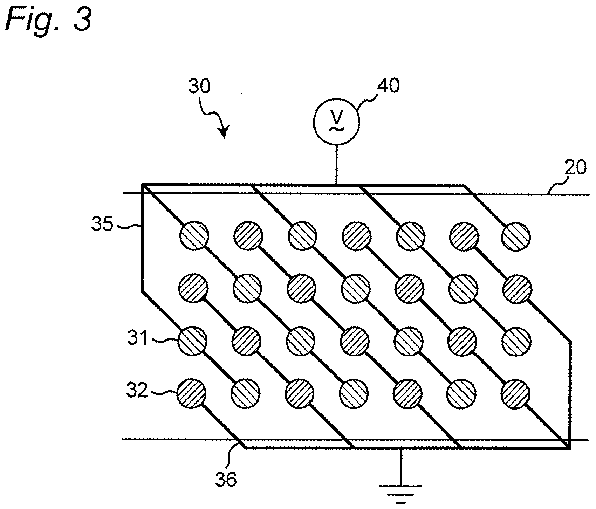

Fig. 2 is a perspective view showing a configuration of the electrode unit 30 of the separation device 1 according to the first embodiment, and Fig. 3 is a plan view showing a configuration of the electrode unit 30 of the separation device 1 according to the first embodiment. The electrode unit 30 includes a plurality of signal electrodes 31, a plurality of ground electrodes 32, signal wiring 35, and ground wiring 36.

Each of the signal electrodes 31 and the ground electrodes 32 is a three-dimensional electrode extending in the height direction of the flow channel 20. In the present disclosure, the three-dimensional electrode is an electrode having a height of at least 10 $\mu$m and does not include a wiring pattern electrode formed by photolithography or the like. In the present embodiment, for example, each of the signal electrodes 31 and the ground electrodes 32 has a cylindrical shape extending in the height direction of the flow channel 20.

The signal electrodes 31 and the ground electrodes 32 are arranged in a matrix form at a predetermined interval in the liquid flow direction and the width direction of the flow channel 20. The predetermined interval is set, for example, such that the ratio of the diameter of the electrodes to the distance between the electrodes is about 60 : 100. In this electrode arrangement, the signal electrodes 31 and the ground electrodes 32 are alternately arranged so that the electrodes adjacent in the liquid flow direction and the width direction are different from each other.

More specifically, the signal electrodes 31 and the ground electrodes 32 are alternately arranged at a substantially equal interval in the liquid flow direction and are alternately arranged at a substantially equal interval in the width direction. The signal electrodes 31 to which the same voltage is applied are arranged at a substantially equal interval in an oblique direction forming an angle of about 45° with respect to the liquid flow direction, similarly, the ground electrodes 32 to which the same voltage is applied are arranged at a substantially equal interval in an oblique direction forming an angle of about 45° with respect to the liquid flow direction. The row of the signal electrodes 31 obliquely at about 45° and the row of the ground electrodes 32 obliquely at about 45° are alternately arranged at a substantially equal interval in the liquid flow direction.

Materials of the signal electrodes 31 and the ground electrodes 32 include metal such as nickel, copper, gold, silver, platinum, zinc, tin, chromium or the like, conductive polymers, carbon nanotubes, or the like. The diameters of the signal electrodes 31 and the ground electrodes 32 are about 5 $\mu$m or more and about 500 $\mu$m or less. The heights of the signal electrodes 31 and the ground electrodes 32 are about 10 $\mu$m or more and about 1000 $\mu$m or less.

The signal wiring 35 is wiring for connecting the plurality of signal electrodes 31 and the power supply 40, and supplying the AC voltage from the power supply 40 to the signal electrodes 31. The ground wiring 36 is wiring for connecting the plurality of ground electrodes 32 to the ground.

2. Operation

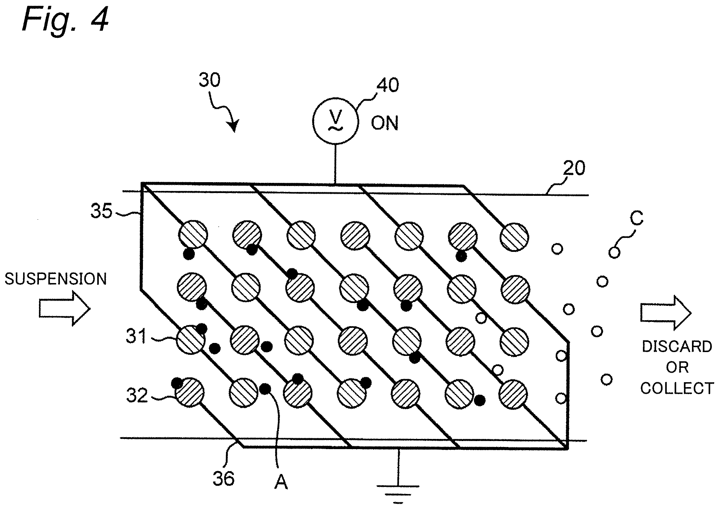

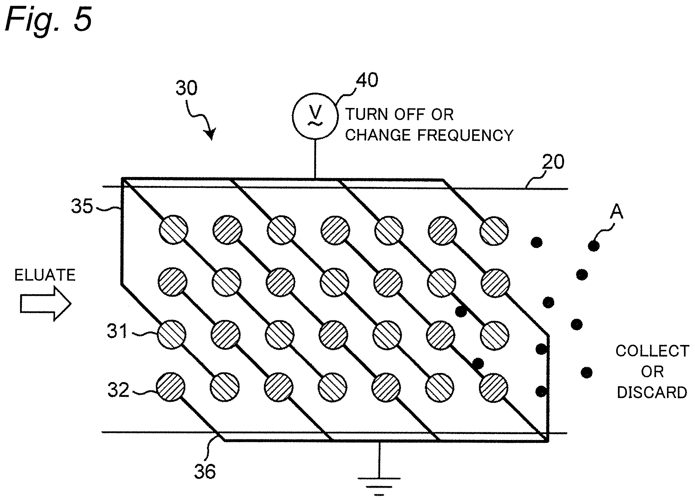

With reference to Figs. 4 and 5, the operation of the separation device 1 configured as described above will be described below.

In the first embodiment, a strong positive dielectrophoretic force (attracting force) is applied to target cells to capture the target cells on the electrodes 31 and 32, and cells other than the target cells are excluded. Thereafter, by stopping the application of the AC voltage between the electrodes 31 and 32 and by flowing the eluate, the target cells captured by the electrodes 31 and 32 are collected to thereby be separated.

First, as shown in Fig. 4, under the control of the controller 50, a suspension containing target cells A and other cells C and a dielectrophoretic liquid are introduced from the liquid introduction unit 10 to the flow channel 20. Also, under the control of the controller 50, an AC voltage with a predetermined frequency from the power supply 40 is applied between the signal electrodes 31 and the ground electrodes 32 of the electrode unit 30.

When the target cells A flowing through the flow channel 20 pass between the signal electrodes 31 and the ground electrodes 32, a positive dielectrophoretic force (attracting force) acts on the target cells A, and the target cells A are attracted to and captured by the signal electrodes 31 and the ground electrodes 32. On the other hand, no dielectrophoretic force acts on the cells C other than the target cells, or even if a positive or negative dielectrophoretic force (attracting force or repulsive force) acts on them, the dielectrophoretic force is relatively small. Therefore, the cells C other than the target cells pass between the signal electrodes 31 and the ground electrodes 32 and are discarded in the collection unit 60.

Next, as shown in Fig. 5, under the control of the controller 50, the eluate is introduced into the flow channel 20 from the liquid introduction unit 10. Further, under the control of the controller 50, the application of the AC voltage between the signal electrodes 31 and the ground electrodes 32 of the electrode unit 30 from the power supply 40 is stopped. As a result, the target cells A captured by the signal electrodes 31 and the ground electrodes 32 are flowed by the eluate and collected by the collection unit 60.

3. Summary

As described above, according to the present embodiment, the electrodes 31 and 32 of the electrode unit 30 form a solid geometry (three-dimensional shape) extending in the height direction of the flow channel 20 through which cells (dielectric particles) flow, even if the height of the flow channel 20 is increased, uniform dielectrophoretic force can be applied to cells passing under the flow channel 20 and to cells passing above the flow channel 20. Therefore, the effective volume of cell separation treatment can be increased, and the separation treatment capacity can be improved.

Further, according to the present embodiment, uniform dielectrophoretic force can be applied to cells passing under the flow channel 20 and to cells passing above the flow channel 20, so that the separation accuracy is high from below to above the flow channel 20.

Further, according to the present embodiment, since the cell-capturing surface is large (the specific surface area is large) by the structure in which many three-dimensional electrodes 31 and 32 are arranged, it is possible to reliably capture cells.

4. Modification

In the first embodiment, a positive dielectrophoretic force (attracting force) is applied to target cells A passing through the electrodes 31 and 32, and the target cells A are attached by the electrodes 31 and 32 to thereby be captured. However, the idea of the present disclosure is not limited to this. A negative dielectrophoretic force (repulsive force) may be applied to target cells A passing through the electrodes 31 and 32, and the target cells A may be captured between the electrodes 31 and 32 by repelling the electrodes 31 and 32.

Further, in the first embodiment, the application of the AC voltage to the electrodes 31 and 32 is stopped, and the target cells A captured by the electrodes 31 and 32 are collected. The present disclosure is not limited thereto. The frequency of the AC voltage applied to the electrodes 31 and 32 is changed to a frequency in which dielectrophoretic force does not act, or even when a positive or negative dielectrophoretic force (attracting force or repulsive force) acts, the dielectrophoretic force becomes relatively small, so that the target cells A captured by the electrodes 31 and 32 may be collected.

In the first embodiment, the target cells A are captured by the electrodes 31 and 32, the cells C other than the target cells are discharged, and then the target cells A captured by the electrodes 31 and 32 are collected. The present disclosure is not limited thereto. A dielectrophoretic force is applied to the cells C other than the target cells to capture the cells C other than the target cells at the electrodes 31 and 32, and the dielectrophoretic force is not applied to the target cells A, or even if the dielectrophoretic force acts, the dielectrophoretic force becomes relatively small, so that the target cells A may be collected.

(Second Embodiment)

In the first embodiment, a strong positive dielectrophoretic force (attracting force) is applied to the target cells to capture the target cells on the electrodes 31 and 32, cells other than the target cells are discarded, and thereafter, the application of the AC voltage between the electrodes 31 and 32 is stopped, so that the target cells captured by the electrodes 31 and 32 are collected and separated. In the second embodiment, a relatively weak positive dielectrophoretic force (attracting force) is applied to the target cells, and by utilizing the fact that the time required for target cells and other cells to pass between the electrodes 31 and 32 is different because the intensity of the dielectrophoretic force between the target cells and the other cells is different, target cells are separated (dielectrophoresis chromatography).

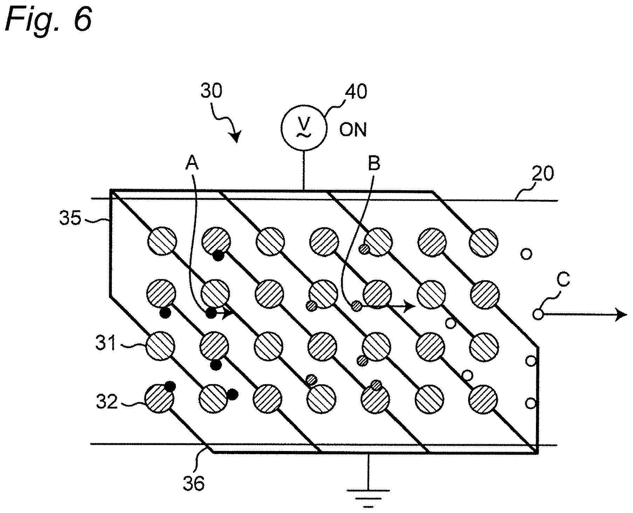

Hereinafter, the operation of the separation device 1 according to the second embodiment will be described with reference to Fig. 6.

Under the control of the controller 50, the suspension containing the target cells A and B and other cells C, and the dielectrophoretic liquid are introduced from the liquid introduction unit 10 into the flow channel 20. Also, under the control of the controller 50, an AC voltage with a predetermined frequency from the power supply 40 is applied between the signal electrodes 31 and the ground electrodes 32 of the electrode unit 30.

Here, it is assumed that the magnitude of dielectrophoretic force (attracting force) acting on each cell A, B, C at a predetermined frequency increases in the order of cells C, cells B, cells A. In addition, the AC voltage is adjusted to a voltage at which the target cells A and B are attracted to the electrodes 31 and 32, and are flowed by the flow of the liquid.

When the cells A, B, C flowing in the flow channel 20 pass between the signal electrodes 31 and the ground electrodes 32, no dielectrophoretic force acts on the cells C other than the target cells, or even if a positive or negative dielectrophoretic force (attracting force or repulsive force) act, the dielectrophoretic force is relatively small. Therefore, the cells C pass between the signal electrodes 31 and the ground electrodes 32 faster than the cells A and B, and are discarded in the collection unit 60.

On the other hand, a positive dielectrophoretic force (attracting force) acts on the target cells A and B, and the target cells A and B are attracted to the signal electrodes 31 and the ground electrodes 32, and are caused to flow by the flow of the liquid. Then, due to the difference in the intensity of the dielectrophoretic force, the target cells B and the target cells A sequentially passes between the signal electrodes 31 and the ground electrodes 32 in the order of the target cells B and the target cells A, and are sequentially collected by the collection unit 60.

As described above, according to the present embodiment, the cell suspension intermittently introduced into the electrode unit 30 serving as a field of separation is discharged from the electrode unit 30 based on the difference in dielectrophoretic force applied to each cell, which is sequentially collected by the collection unit 60. Since the intensity of the dielectrophoretic force is due to the difference in electrical characteristics arising from the difference in the cell structure, it is possible to separate each kind of cells by collecting by time at the collection unit 60.

Modification

In the present embodiment, in order to adjust the intensity of the dielectrophoretic force acting on the cells, the magnitude of the AC voltage applied between the electrodes 31 and 32 is adjusted. The present disclosure is not limited to this. For example, by repeatedly supplying and stopping AC voltage to the electrodes 31 and 32, the application time of the AC voltage to the electrodes 31 and 32 is adjusted, so that the intensity of the dielectrophoretic force acting on the cells may be adjusted.

(Third Embodiment)

In the first embodiment, the signal electrodes 31 and the ground electrodes 32 of the electrode unit 30 are arranged in a matrix in the liquid flow direction and the width direction of the flow channel 20. In the third embodiment, the signal electrodes 31 and the ground electrodes 32 of the electrode unit 30 are arranged in a substantially straight line in an oblique direction forming a predetermined angle with respect to the liquid flow direction of the flow channel 20.

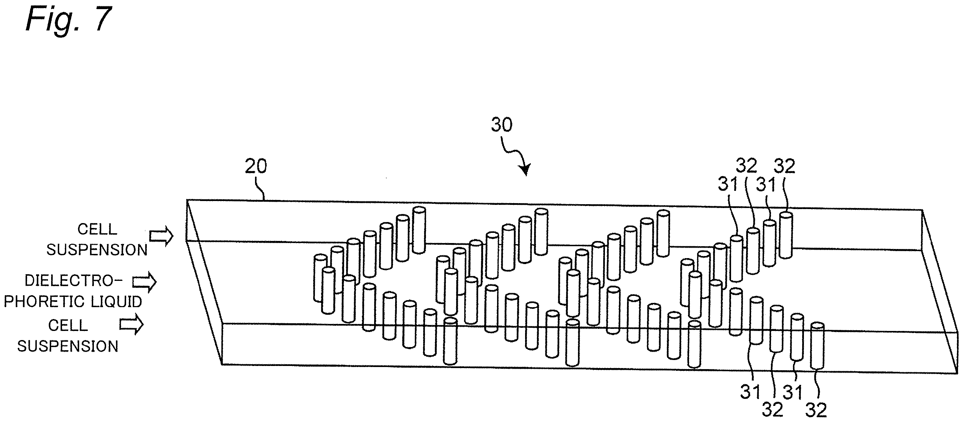

Hereinafter, the electrode unit of the separation device according to the third embodiment will be described with reference to Figs. 7 to 10.

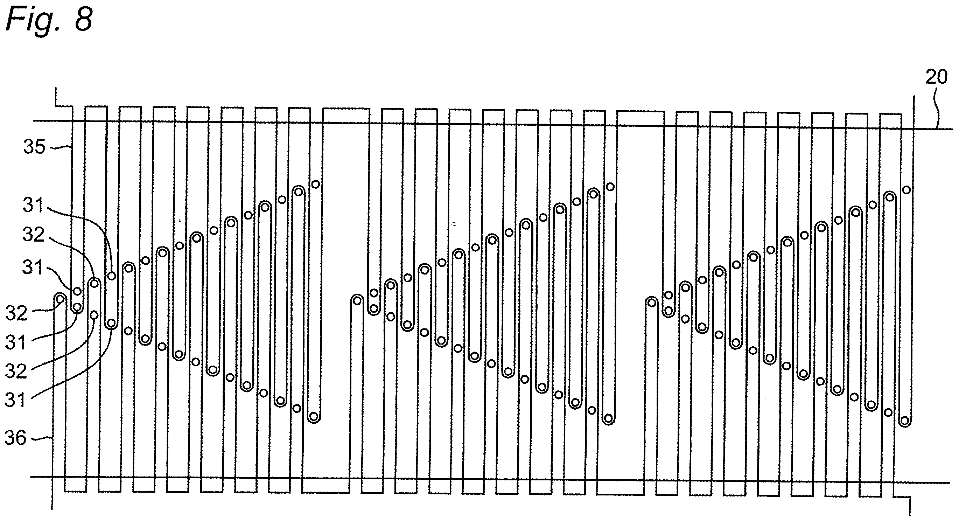

Fig. 7 is a perspective view showing a configuration of the electrode unit of the separation device according to the third embodiment, and Fig. 8 is a plan view showing a configuration of the electrode unit of the separation device according to the third embodiment.

The signal electrodes 31 and the ground electrodes 32 of the electrode unit 30 according to third embodiment are arranged in an oblique direction forming an angle of about 5° or more and 80° or less with respect to the liquid flow direction of the flow channel 20 at a substantially predetermined regular interval and in a substantially straight line. In the present embodiment, the signal electrodes 31 and the ground electrodes 32 are arranged in a substantially V shape from the central portion in the width direction of the flow channel 20 toward both side portions. In this arrangement, the signal electrodes 31 and the ground electrodes 32 are arranged so that the electrode interval in the width direction of the flow channel 20 is substantially constant from the upstream to the downstream of the flow channel 20. In this case, the electrode interval in the liquid flow direction is substantially constant. In this arrangement, the signal electrodes 31 and the ground electrodes 32 are alternately arranged so that adjacent electrodes are different from each other. It should be noted that a plurality of stages of electrode pairs may be arranged in the liquid flow direction of the flow channel 20 with these electrodes arranged in a form of a substantially V-shape as one set.

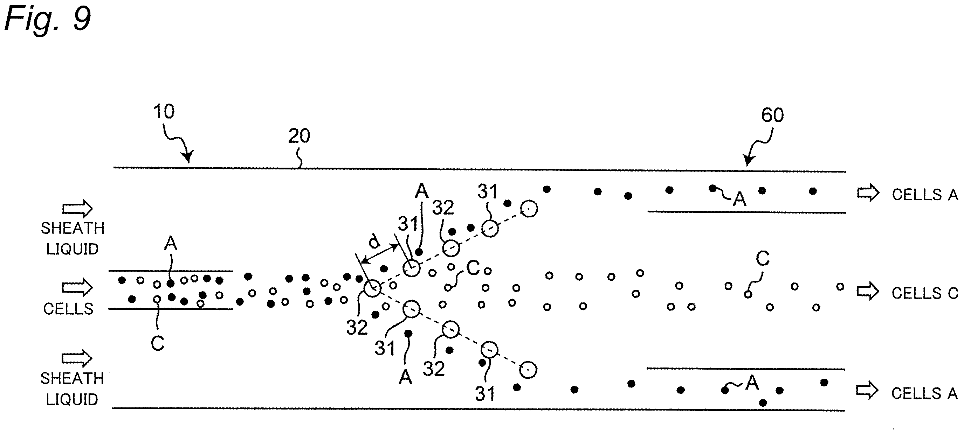

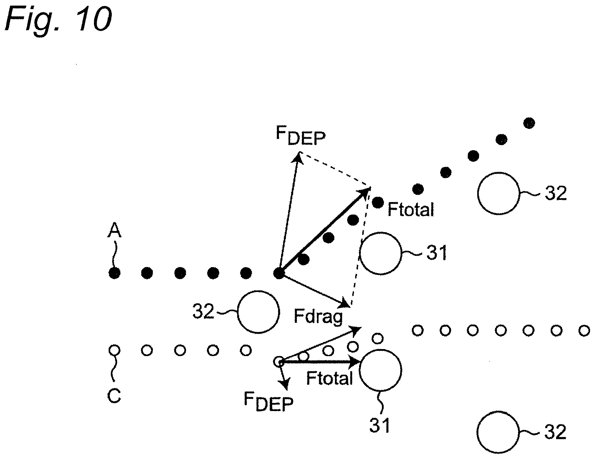

Hereinafter, the operation of the separation device 1 according to the third embodiment will be described with reference to Figs. 9 and 10.

In the third embodiment, a negative dielectrophoretic force (repulsive force) is applied to target cells to obliquely move the target cells along the electrodes 31 and 32 while repelling the target cells against the electrodes 31 and 32, so that the target cells are collected at the side of the flow channel 20 and separated.

As shown in Fig. 9, under the control of the controller 50, a suspension containing target cells A and other cells C is introduced from the liquid introduction unit 10 to the central portion in the width direction of the flow channel 20, and the dielectrophoretic liquid is introduced from the liquid introduction unit 10 to a portion other than the central portion in the width direction of the flow channel 20. Also, under the control of the controller 50, an AC voltage with a predetermined frequency from the power supply 40 is applied between the signal electrodes 31 and the ground electrodes 32 of the electrode unit 30.

When the target cells A and the other cells C flow through the central portion of the flow channel 20 and reach the electrode located in the central portion, a negative dielectrophoretic force (repulsive force) acts on the target cells A, and the target cells A repel the signal electrodes 31 and the ground electrodes 32 and flows obliquely along these electrodes 31 and 32, reach the side portion of the flow channel 20, and are collected at the side portion of the collection unit 60. On the other hand, the dielectrophoretic force does not act on the cells C other than the target cells, or even if the positive or negative dielectrophoretic force (attracting force or repulsive force) acts, the dielectrophoretic force is relatively small. Therefore, the cells C other than the target cells pass between the signal electrodes 31 and the ground electrodes 32, flow through the central portion of the flow channel 20, and are discarded at the central portion of the collection unit 60.

Fig. 10 shows the force received by the cells in the vicinity of the electrodes 31 and 32 and the movement trajectory of the cells. Cells are subjected to the force by fluid flow and dielectrophoretic force. Although the force changes depending on the position from the electrode, when the resultant force acts so that the cells do not pass between the electrodes 31 and 32, the cells are obliquely moved along the electrodes 31 and 32 while repelling the electrodes 31 and 32, whereby the target cells can be collected at the side portion of the flow channel 20 and separated. Therefore, the dielectrophoretic force is controlled by adjusting the voltage and the frequency, and the force received by the flow is controlled by adjusting the volume flow rate of the suspension containing the cells.

As described above, according to the present embodiment, depending on the relationship between the force received by the cells by the flow and the force of the dielectrophoretic force, it is determined whether the cells pass between the electrodes 31 and 32 or move obliquely along the electrodes 31 and 32, and displacement occurs at the collection unit 60, so that the cells are separated by the difference in dielectrophoretic force. Therefore, it is possible to extract only specific target cells from the cell suspension. In the present embodiment, the type and state of the cells contained in the cell suspension are not limited to two types. One or more specific types of cells from three or more types can be separated at the collection unit. Furthermore, by employing a configuration of two or more stages in which the cells acquired by the collection unit 60 are again put into the separation device, it is possible to perform separation according to a variety of cell types and cell states.

Modification

In the present embodiment, the signal electrodes 31 and the ground electrodes 32 are arranged in a substantially V shape from the central portion in the width direction of the flow channel 20 toward both side portions. The present disclosure is not limited to this, and the electrodes may be arranged in a substantially inverted V shape from both side portions in the width direction of the flow channel 20 toward the central portion. In this case, a suspension containing target cells may be introduced into both side portions in the width direction of the flow channel 20.

Further, the signal electrodes 31 and the ground electrodes 32 may be arranged in a substantially straight line from one side portion toward the other side portion in the width direction of the flow channel 20. In this case, the suspension containing the target cells may be introduced from one side portion in the width direction of the flow channel 20.

(Fourth Embodiment)

In the third embodiment, the signal electrodes 31 and the ground electrodes 32 of the electrode unit 30 are arranged at a substantially equal interval. In the fourth embodiment, the signal electrodes 31 and the ground electrodes 32 of the electrode unit 30 are arranged such that the electrode intervals become gradually wider from the central portion to the side portion of the flow channel 20 as the electrodes are arranged closer to the side portion.

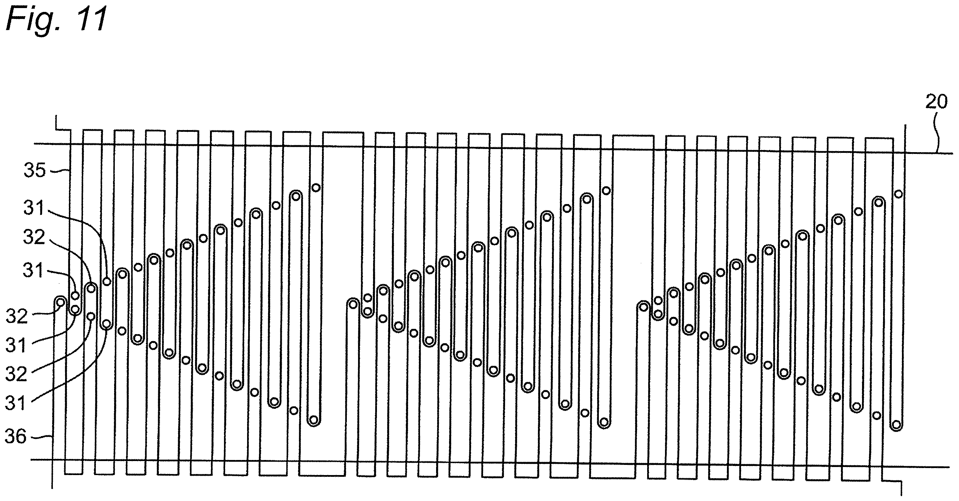

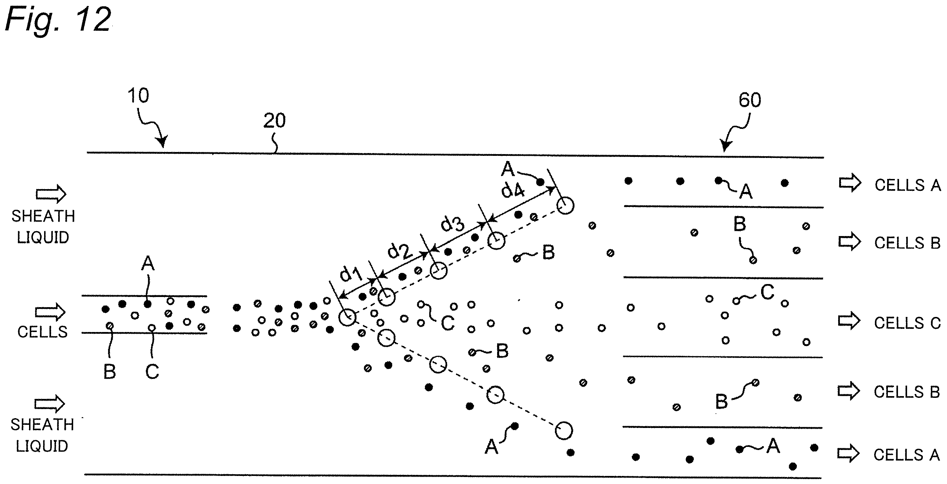

Hereinafter, the electrode unit of the separation device according to the fourth embodiment will be described with reference to Figs. 11 and 12.

Fig. 11 is a plan view showing a configuration of an electrode unit of the separation device according to the fourth embodiment. In the third embodiment, in the substantially V-shaped arrangement of the signal electrodes 31 and the ground electrodes 32, the signal electrodes 31 and the ground electrodes 32 are arranged so that the electrode intervals in the width direction of the flow channel 20 increases by degrees from the upstream toward the downstream of the flow channel 20. In this case, the amount of change in the electrode interval in the liquid flow direction and the amount of change in the electrode interval in the width direction are constant. The amount of change in the electrode interval in the liquid flow direction and the amount of change in the electrode interval in the width direction may not be constant.

Hereinafter, the operation of the separation device 1 according to the fourth embodiment will be described with reference to Fig. 12.

Under the control of the controller 50, a suspension containing target cells A and B and other cells C is introduced from the liquid introduction unit 10 to the central portion in the width direction of the flow channel 20, and the dielectrophoretic liquid is introduced from the liquid introduction unit 10 to a portion other than the central portion in the width direction of the flow channel 20. Also, under the control of the controller 50, an AC voltage with a predetermined frequency from the power supply 40 is applied between the signal electrodes 31 and the ground electrodes 32 of the electrode unit 30.

Here, it is assumed that the magnitude of dielectrophoretic force (repulsive force) acting on each cell A, B, C at a predetermined frequency increases in the order of cells C, cells B, cells A. Further, the AC voltage is adjusted to such a voltage that the target cells A and B repel the electrodes 31 and 32 and obliquely flow along the arrangement direction of the electrodes 31 and 32 by the flow of the liquid.

When the cells A, B, C flowing in the flow channel 20 pass between the signal electrodes 31 and the ground electrodes 32, no dielectrophoretic force acts on the cells C other than the target cells, or even if a positive or negative dielectrophoretic force (attracting force or repulsive force) acts on them, the dielectrophoretic force is relatively small. Therefore, the cells C linearly pass between the signal electrodes 31 and the ground electrodes 32, flow through the central portion, and are discarded at the central portion of the collection unit 60.

On the other hand, a negative dielectrophoretic force (repulsive force) acts on the target cells A and B, and the target cells A and B obliquely flow along the electrodes 31 and 32 while repelling the signal electrodes 31 and the ground electrodes 32. Then, due to the difference in the intensity of the dielectrophoretic force, the target cells A reach the side portion of the flow channel 20, while the target cells B pass between the signal electrodes 31 and the ground electrodes 32 at the middle portion between the central portion and the side portion of the flow channel 20. Then, the target cells B linearly flow in the middle portion of the flow channel 20, and are collected at the middle portion between the central portion and the side portion of the collection unit 60. On the other hand, the target cells A are collected at the side portion of the collection unit 60.

As described above, according to the present embodiment, depending on the relationship between the force received by the cells by the flow and the force of the dielectrophoretic force, it is determined whether the cells pass between the electrodes 31 and 32 or move obliquely along the electrodes 31 and 32, and displacement occurs at the collection unit 60, so that the cells are separated by the difference in dielectrophoretic force. As the distance between the electrodes 31 and 32 increases, the electric field intensity generated between the electrodes decreases, so that the dielectrophoretic force applied to the cells decreases. Therefore, cells pass sequentially between the electrodes 31 and 32 in the order of the cells to which weaker dielectrophoretic force is applied. Cells to which the dielectrophoretic force is not applied or sufficiently weakly applied are collected at the central portion of the collection unit, and as the dielectrophoretic force increases, cells to which the dielectrophoretic force is strongly applied are collected at the side portion of the collection unit rather than the central portion. Since the intensity of the dielectrophoretic force is caused by a difference in electrical characteristics arising from a difference in cell structure, displacement occurs for the collection unit depending on the type and state of the cells, and it is possible to fractionate the cells. In the present embodiment, the type and state of the cells contained in the cell suspension are not limited to three, and a large number of electrode pairs having different electrode distances can be prepared and separated according to a variety of cell types and cell states by fractionating them in large numbers for each displacement by the collection unit. Furthermore, it is also possible to employ a configuration of two or more stages in which the cells acquired by the collection unit 60 are again put into the separation device.

Modification

Also in this embodiment, the signal electrodes 31 and the ground electrodes 32 may be arranged in substantially inverted V shape from both side portions in the width direction of the flow channel 20 toward the central portion. In this case, the signal electrodes 31 and the ground electrodes 32 may be arranged so that the electrode intervals gradually increase toward the central portion from the side portion.

Further, the signal electrodes 31 and the ground electrodes 32 may be arranged in a substantially straight line from one side portion toward the other side portion in the width direction of the flow channel 20. In this case, the signal electrodes 31 and the ground electrodes 32 may be arranged so that the electrode intervals gradually increase from one side portion toward the other side portion.

(Other Embodiments)

In the first to fourth embodiments described above, bacteria and cells are exemplified as a separation target of the present apparatus. The separation target of the present apparatus is not limited to bacteria and cells. It may be any dielectric particles, and may be, for example, microorganisms, fungi, spores, viruses, DNA, RNA, carbon nanotubes, emulsions, and microcapsules.

Further, in the first to fourth embodiments described above, a separation device that separates dielectric particles has been described. The spirit of the present disclosure is not limited thereto, and may be applied to a concentration apparatus or the like that concentrates liquid containing dielectric particles.

Further, in the above-described first to fourth embodiments, the cylindrical electrodes 31 and 32 are illustrated. The shape of the electrode according to the present disclosure is not limited to a cylindrical shape. It may be any three-dimensional shape, for example, a polygonal shape, a conical shape, or a polygonal prism shape.

**Claims**

1. A separation device (1) for separating dielectric particles (A, B, C), comprising:

   a flow channel (20) configured to feed a suspension containing the dielectric particles (A, B, C);
   a plurality of three-dimensionally shaped electrodes (31, 32) arranged in the flow channel (20) and extending in a height direction of the flow channel (20);
   a power supply (40) configured to apply an AC voltage with a predetermined frequency to the plurality of electrodes (31, 32) so as to generate dielectrophoresis of the dielectric particles (A, B, C); and
   a controller (50) configured to control the power supply (40), **characterized in that**
   the plurality of the electrodes (31, 32) are arranged in an oblique direction forming a predetermined angle with respect to a liquid flow direction, with arrangement of the plurality of electrodes (31, 32) being a V-shape convex on upstream or downstream of the liquid flow direction.

2. The separation device (1) according to claim 1, wherein

   the plurality of electrodes (31, 32) include signal electrodes (31) to which the AC voltage is applied and ground electrodes (32) connected to the ground, and
   the signal electrodes (31) and the ground electrodes (32) are alternately arranged so that electrodes (31, 32) adjacent in an arrangement direction are different from each other.

3. The separation device (1) according to claim 1 or 2, wherein when feeding, to the flow channel (20), an eluate for delivering out the dielectric particles (A, B C) captured by the plurality of electrodes (31, 32) from the flow channel (20), the controller (50) is configured to cause the power supply (40) to stop applying an AC voltage to the plurality of electrodes (31, 32).

4. The separation device (1) according to claim 1 or 2, wherein the controller (50) is configured to adjust a magnitude or an application time of an AC voltage applied to the plurality of electrodes (31, 32) so as to adjust a magnitude of dielectrophoretic force acting on the dielectric particles (A, B, C).

5. The separation device (1) according to claim 1, wherein electrode intervals ($d_1$, $d_2$, $d_3$, $d_4$) of the plurality of electrodes (31, 32) increase gradually in an arrangement direction.

**Patentansprüche**

1. Trennvorrichtung (1) zum Trennen von dielektrischen Partikeln (A, B, C) mit:

   einem Flusskanal (20), der ausgestaltet ist, eine Suspension mit den dielektrischen Partikeln (A, B, C) zu führen,
   mehreren dreidimensional geformten Elektroden (31, 32), die in dem Flusskanal (20) angeordnet sind und sich in einer Höhenrichtung des Flusskanals (20) erstrecken,
   einer Leistungszufuhr (40), die ausgestaltet ist, an die mehreren Elektroden (31, 32) eine Wechselspannung mit einer vorbestimmten Frequenz anzulegen, um eine Dielektrophorese der dielektrischen Partikel (A, B, C) zu erzeugen, und
   einer Steuereinheit (50), die ausgestaltet ist, die Leistungszufuhr (40) zu steuern, **gekennzeichnet dadurch, dass**
   die mehreren Elektroden (31, 32) in einer schrägen Richtung angeordnet sind, die einen vorbestimmten Winkel hinsichtlich einer Flüssigkeitsfließrichtung bildet, mit einer Anordnung der mehreren Elektroden (31, 32) in einer V-Form konvex stromaufwärts oder stromabwärts in der Flüssigkeitsfließrichtung.

2. Trennvorrichtung (1) nach Anspruch 1, wobei

   die mehreren Elektroden (31, 32) Signalelektroden (31), an die die Wechselspannung angelegt wird, und Masseelektroden (32) umfasst, die mit Masse verbunden sind, und
   die Signalelektroden (31) und die Masseelektroden (32) abwechselnd angeordnet sind, sodass sich Elektroden (31, 32), die Anordnungsrichtung benachbart sind, voneinander unterscheiden.

3. Trennvorrichtung (1) nach Anspruch 1 oder 2, wobei wenn dem Flusskanal (20) ein Eluat zum Ausliefern der dielektrischen Partikel (A, B, C), die durch die mehreren Elektroden (31, 32) aus dem Flusskanal (20) zugeführt wird, die Steuereinheit (50) ausgestaltet ist, die Leistungszufuhr (40) zu veranlassen, das Anlegen einer Wechselspannung an die mehreren Elektroden (31, 32) zu stoppen.

4. Trennvorrichtung (1) nach Anspruch 1 oder 2, wobei die Steuereinheit (50) ausgestaltet ist, eine Stärke oder eine Anlegezeit der Wechselspannung, die an die mehreren Elektroden (31, 32) angelegt wird, zu justieren, um so eine Stärke der dielektrophoretischen Kraft zu justieren, die auf die dielektrischen Partikel (A, B, C) wirkt.

5. Trennvorrichtung (1) nach Anspruch 1, wobei Elektrodenintervalle ($d_1$, $d_2$, $d_3$, $d_4$) der mehreren Elektroden (31, 32) in einer Anordnungsrichtung graduell zunehmen.

**Revendications**

1. Dispositif de séparation (1) pour séparer des particules diélectriques (A, B, C), comprenant :

un canal d'écoulement (20) configuré pour écouler une suspension contenant les particules diélectriques (A, B, C) ;
une pluralité d'électrodes de forme tridimensionnelle (31, 32) disposées dans le canal d'écoulement (20) et s'étendant dans une direction de hauteur du canal d'écoulement (20) ;
une alimentation électrique (40) configurée pour appliquer une tension alternative à une fréquence prédéterminée à la pluralité d'électrodes (31, 32) pour générer une diélectrophorèse des particules diélectriques (A, B, C), et
un contrôleur (50) configuré pour contrôler l'alimentation électrique (40), **caractérisée en ce que**
la pluralité d'électrodes (31, 32) est disposée dans une direction oblique formant un angle prédéterminé par rapport à une direction d'écoulement du liquide, la disposition de la pluralité d'électrodes (31, 32) étant en forme de V convexe en amont ou en aval du sens d'écoulement du liquide.

2. Dispositif de séparation (1) selon la revendication 1, dans lequel

la pluralité d'électrodes (31, 32) comprend des électrodes de signal (31) auxquelles la tension alternative est appliquée et des électrodes de masse (32) qui sont connectées à la masse, et
les électrodes de signal (31) et les électrodes de masse (32) sont disposées alternativement de telle sorte que les électrodes (31, 32) adjacentes les unes aux autres dans la direction de la disposition sont différentes les unes des autres.

3. Dispositif de séparation (1) selon la revendication 1 ou 2, dans lequel lorsqu'un éluat pour délivrer les particules diélectriques (A, B, C) fournies par les multiples électrodes (31, 32) du canal d'écoulement (20) est fourni au canal d'écoulement (20), le contrôleur (50) est configuré pour faire en sorte que l'alimentation électrique (40) cesse d'appliquer une tension alternative aux multiples électrodes (31, 32).

4. Dispositif de séparation (1) selon la revendication 1 ou 2, dans lequel le contrôleur (50) est configuré pour ajuster une magnitude ou un temps d'application de la tension alternative appliquée à la pluralité d'électrodes (31, 32) de façon à ajuster une magnitude de la force diélectrophorétique agissant sur les particules diélectriques (A, B, C).

5. Dispositif de séparation (1) selon la revendication 1, dans lequel les intervalles d'électrodes (d1, d2, d3, d4) de la pluralité d'électrodes (31, 32) augmentent progressivement dans une direction d'agencement.

Fig. 1

Fig. 2

Fig. 3

*Fig. 4*

30

40
ON

20

C

DISCARD
OR
COLLECT

35

31

32

36

A

SUSPENSION

*Fig. 5*

COLLECT OR DISCARD

A

20

TURN OFF OR CHANGE FREQUENCY

40

30

35

31

32

36

ELUATE

Fig. 6

Fig. 7

Fig. 8

EP 3 418 373 B1

Fig. 9

EP 3 418 373 B1

Fig. 10

Fig. 11

20

35 31 32 31 32 31 32 31 36

Fig. 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007152206 A1
- US 2008283401 A1
- WO 2013019810 A2
- WO 2012054904 A2
- WO 0105512 A1
- JP 2013027366 A